# EUROPEAN PATENT SPECIFICATION

(11) **EP 1 250 165 B1**
(45) Date of publication and mention of the grant of the patent: **13.05.2009**
(21) Application number: 01906708.1
(22) Date of filing: 25.01.2001
(51) Int. Cl.: A61L 27/54, A61L 33/00

(54) **BIOACTIVE COATINGS TO PREVENT TISSUE OVERGROWTH ON ARTIFICIAL HEART VALVES**
BIOAKTIVE BESCHICHTUNGEN ZUR VERMEIDUNG VON GEWEBEWACHSTUM AUF KÜNSTLICHEN HERZKLAPPEN
REVETEMENTS BIOACTIFS DE PREVENTION DE LA PROLIFERATION TISSULAIRE SUR DES VALVULES CARDIAQUES ARTIFICIELLES

(30) Priority: 25.01.2000 US 178084 P; 16.05.2000 US 571987
(43) Date of publication of application: 23.10.2002
(73) Proprietor: Edwards Lifesciences Corporation, Irvine, CA 92614 (US)
(72) Inventor: HELMUS, Michael, N., Massachussets 06109 (US); CUNANAN, Crystal, Mission Viejo, CA 92691 (US); TREMBLE, Patrice, Santa Rosa, CA 95403 (US); KAFESJIAN, Ralph, Newport Beach, CA 92663 (US)
(74) Representative: Parry, Christopher Stephen
(86) International application number: PCT/US2001/002621
(87) International publication number: WO 2001/054745

(56) References cited:
- WO-A-95/07691
- WO-A-96/30060
- US-A- 5 925 552
- US-A- 6 007 577

## Description

### BACKGROUND OF THE INVENTION

Currently available prostheses for the replacement of defective heart valves and other vascular structures may be classified as mechanical or bioprosthetic. Mechanical heart valves are manufactured from biocompatible materials, including metals and materials such as Silastic®, graphite titanium and Dacron®. The valves are typically comprised of a sewing ring and a housing unit containing the valve leaflets and occluder. Representative mechanical heart valves are disclosed in, for example, Olin, U.S. Patent No. 4,863,459, issued September 5, 1989; Hansen et al., U.S. Patent No. 4,276,658, issued July 7, 1981; and Bokros, U.S. Patent No. 4,689,046, issued August 25, 1987. Although mechanical valves have the advantage of proven durability, they are frequently associated with a high incidence of blood clotting on or around the valve. For this reason, patients with implanted mechanical valves generally must remain on anticoagulants for as long as the valve remains implanted. Moreover, these devices can become overgrown with host fibrous tissue ("pannus"), which can deleteriously affect the performance of the valves.

Bioprosthetic valves were introduced in the early 1960's and are typically derived from pig aortic valves or are manufactured from other biological materials such as bovine pericardium. Xenograft heart valves invariably are tanned in glutaraldehyde prior to implantation. A major rationale for the use of biological material for heart valves is that the profile and surface characteristics of this material are optimal for laminar, nonturbulent blood flow. The result is that intravascular clotting is less likely to occur than with mechanical valves. This concept has been clinically proven with the well-documented reduced thrombogenicity of current versions of glutaraldehyde-fixed heart valves (*see*, for example, Yang et al., U.S. Patent No. 5,935,168, issued August 10, 1999; and Gross, U.S. Patent No. 5,824,065, issued October 20, 1998.)

In both mechanical and bioprosthetic heart valves, excessive clotting remains a serious concern. The thrombus produced by clotting can lead to acute or subacute closure of the valve. Moreover, this thrombus becomes the matrix for wound healing and incorporation of fibrous tissue into the structure of the heart valve. The excess fibrous tissue, i.e., pannus, can form on one or more component of the valve. When a mechanical valve is utilized, the pannus can grow on the sewing ring, housing and/or the valve leaflets and or the occluder within the housing component. The situation is similar with a bioprosthetic valve.

With a bioprosthetic valve, the pannus can grow onto any or all of the components, including the tissue leaflets. The growth of pannus on the leaflets can cause the leaflets to stiffen, thereby leading to a loss of valve function. Moreover, the pannus can grow over the fabric at the cusp cover of the stent and interfere with the proper coaptation of the leaflets. Interference with the normal functioning of either a bioprosthetic or mechanical valve can reduce its performance to such an extent that it must be replaced, subjecting the recipient to another major surgical procedure. At its most extreme, the function of the valve can be so impaired as to cause its failure.

A heart valve designed to prevent or reduce the infiltration of pannus into its structure would have a longer useful lifetime and would offer the recipient of such a prosthetic a better quality of life by diminishing the problems associated with premature valve failure. Towards this end, Tweden has prepared a prosthetic heart valve in which the polyester sewing cuffs were coated with a surface active synthetic protein peptide modeled after the cell attachment domain of fibronectin (PepTite™, Telios Pharmaceuticals, Inc.), Tweden et al., J. Heart Valve Disease 4: (Suppl. I) S90-97 (1995). These workers demonstrated that the peptide coating promoted cell attachment to the fabric cuffs and that the coating was resistant to removal at 37 °C in both saline and plasma over a period of one week.

The RGD peptides utilized by Tweden *et al*. operate by promoting cellular adhesion. Agents preventing the ingrowth of excess pannus by any other mechanism are not disclosed by Tweden *et al*. Moreover, the RGD peptides remain substantially anchored to the fabric of the sewing cuff, forming a foundation for new tissue growth. The peptides apparently do not diffuse, or otherwise penetrate into the surrounding tissue. As the peptides are not administered to the surrounding tissue, there is no provision for any control of the effect of those peptides on the tissue surrounding the sewing cuff. Thus, the peptide can interact with the surrounding tissue in a non-ideal or an undesirable manner, such as in an initial high dosage burst upon installation of the prosthetic valve.

A prosthetic heart valve incorporating an antiproliferative agent in a controlled release format, would offer a number of advantages over a device onto which a promoter of cellular adhesion was simply coated. For example, the large number of known antiproliferative agents allows for great flexibility in the design of the device/bioactive agent construct. Moreover, the controlled release format allows the dosage rate of the agent to be manipulated to produce a release rate appropriate for the agent utilized. As approximately one-half of all emboli occurring are clustered in the first 6-12 postoperative weeks following valve replacement, it is desirable to have a prosthetic valve that actively regulates the formation of thrombus for at least this length of time.

Quite surprisingly, the present invention provides such a heart valve.

WO 96/30060 discloses a cardiac, coronary or vascular prosthesis being coated or impregnated with a hydrophilic polymer composition which may contain a pharmaceutically active agent, for example an anti-coagulant, a thrombolytic agent or an antibiotic. The pharmaceutically active agent may be chemically bound to the hydrophilic polymer or may be physically entrapped within the polymer. There may be more than one layer of hydrophilic polymer coating.

### SUMMARY OF THE INVENTION

It has now been discovered that heart valves incorporating biologically active agents capable of preventing tissue overgrowth have a decreased level of infiltration by recipient-derived fibrous tissue. The agents incorporated into the valves of the invention prevent excess fibrous tissue growth on components of the valve, preferably without substantially impeding tissue ingrowth which is desirably present to cover the exposed fabric of the sewing ring and to anchor the valve to the surrounding tissue. Thus, the present heart valves, whether of mechanical or bioprosthetic design, have fewer problems associated with their use and are generally better tolerated in patients than analogous devices that do not incorporate such biologically active agents.

The present invention provides the prosthetic heart valve of independent claim 1. The dependent claims specify preferred but optional features.

The prosthetic heart valve is resistant to tissue overgrowth following the implantation of the prosthetic heart valve into a host. The prosthetic valves of the invention comprise a sewing ring, typically constructed of a fabric, and a housing component for the leaflets. The sewing ring, the housing component, the valve components within the housing component or a combination thereof comprise a biologically active material that prevents or reduces tissue overgrowth.

### DETAILED DESCRIPTION OF THE INVENTION AND THE PREFERRED EMBODIMENTS

### A. Introduction

The present invention provides prosthetic heart valves comprising biologically active materials or agents that arrest or retard the accumulation of pannus on the prosthetic device itself and in the area adjacent to its implantation in a host. The advantages of the devices of the invention are far-reaching. As the devices of the invention eliminate, or reduce, one of the major factors underlying prosthetic valve failure, these devices hold the premise to improve both the quality and the length of life of the patients receiving these devices.

The present invention provides a prosthetic heart valve resistant to tissue overgrowth following the implantation of the prosthetic heart valve into a host. The prosthetic valves of the invention comprise a sewing ring, typically constructed of a fabric, and a housing component for the leaflets, wherein an antimicrotubule agent, in an amount sufficient to prevent tissue overgrowth, is incorporated into the sewing ring.

The various components of the devices of this aspect of the invention are set forth in greater detail below. The discussion of the components of the device is also applicable to the method of the invention. The following discussion is intended to be illustrative of the scope and breadth of the invention and should not be construed as limiting. Those of skill in the art will appreciate that, with the guidance provided herein, other aspects and embodiments that are within the scope of the invention can be developed.

### B. Structure and Materials

The following discussion is generally applicable to both mechanical and bioprosthetic valves. The heart valves and the sewing rings of the present invention can be made of substantially any material including, but not limited, to metal, ceramics, composites, biologically generated materials and polymeric materials. Appropriate materials falling within these generic designations, as well as other classes of materials, will be apparent to those of skill in the art.

For components of, or within, the valve housing, preferred materials include, for example, machined or cast materials such as pyrolytic carbon and metals. Preferred metals include, but are not limited to, cobalt-chrome alloys, titanium and titanium alloys.

Preferred materials for the sewing ring are those that are biocompatible, strong, durable and flexible. The term "flexible" material generally refers to a material that conforms to the shape of the annulus of the prosthetic valve and the remains of the papillary heads. The term "strong" generally refers to materials that are suturable.

In a presently preferred embodiment, the prosthetic valve of the invention has a sewing ring, a housing component and valve components located within the housing component. Any one, or all of these components are fabricated from a metal, pyrolytic carbon, a polymeric material, or a combination thereof. One or more of the various components of the valve (e.g., housing, leaflets, sewing ring, *etc.)* can also be coated with a polymeric material, a textile or a combination thereof.

When polymeric materials are used to fabricate the valve, or a component of the valve, these materials will generally include one or more materials comprising a member selected from plastics, rubbers and combinations thereof.

Preferred materials include thermoplastic polyurethanes TPUs, nylons, polypropylene, polyoxymethylene, poly(ethylene ketone), polytetrafluoroethylene (PTFE), expanded polytetrafluoroethylene (ePTFE), polyesters, nylon polymers, block copolymers of a polyether polymer and a polyester polymer, and block copolymers of a polyether polyol and one or more members selected from the group consisting of polyamides, polyimides, polyolefins, synthetic hydrocarbon elastomers, and natural rubber.

Even more preferred are materials such as the polyester textile made by Bard Vascular System and having a thickness of about 0.25 mm, polytetrafluoroethylene (PTFE) and expanded polytetrafluoroethylene (ePTFE) under the brandname Teflon® and GoreTex®, respectively, and nylon-11 and -12, polyethylenes (PE) and polypropylenes (PP).

The housing component will typically be constructed in the form of a hard, molded casing enclosing the functional components of the valve. Similarly, the sewing ring can be in the form of an inflexible molded material. Preferably, however, the sewing ring is a flexible structure, which even more preferably includes a polymeric material that is in the form of a fabric. Substantially any type of fabric weave can be used, including, for example, fabrics that are selected from a weft knit with a velour, a weft knit without a velour, a warp knit with a velour, a warp knit without a velour, a weave structure with a velour, a weave structure without a velour and combinations thereof.

The sewing ring for the heart valve can be fabricated from a fabric comprising yarn manufactured from only a single polymeric component. Alternatively, the sewing ring can be fabricate from a combination yarn that includes two or more polymeric components. In those embodiments in which two or more polymeric components are used, it is generally preferred that the combination yarn comprises polyester wrapped with polypropylene yarn. In preferred embodiments, the sewing ring is a commercially available sewing ring, however, the use of non-commereially available sewing rings is contemplated within the scope of the invention. Materials from which non-commercially available sewing rings are fabricated include, for example, porous materials and nylons-11 and -12.

The above discussion is intended to be illustrative, and not limiting, of the range of materials useful for forming prosthetic valves. Other materials useful for manufacturing the various components of a prosthetic valve will be apparent to those of skill in the art

### C. Bioactive Agents

Agents affecting microtubule polymerisation, structure and function that are capable of retarding or arresting pannus infiltration into the implanted prosthetic valves are appropriate for incorporation into the device of the invention.

The antimicrotubule agent is preferably selected from taxane, a derivative of taxane or a combination thereof.

Pharmaceutically acceptable salts of the biologically active agents are also of use in the present invention. Exemplary salts include the conventional non-toxic salts of the compounds of this invention as formed, *e*.*g*., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, trifluoroacetic and the like.

Other agents that are useful in conjunction with the present invention will be readily apparent to those of skill in the art.

### D. Incorporation of Bioactive Agents

The bioactive agents useful in practicing the present invention can be incorporated into the devices of the invention using one or more of the many art-recognized techniques for immobilizing, or adhering, drug molecules to other molecules and surfaces. These methods include, but are not limited to, covalent attachment to the device of the drug or a derivative of the drug bearing a "handle" allowing it to react with a component of the device having a complementary reactivity. Moreover, the bioactive agent can be incorporated into the device using a non-covalent interaction, such as an electrostatic or an ionic attraction between a charged drug and a component of the device bearing a complementary charge. The devices can also be fabricated to incorporate the drugs into reservoirs located on the device or on a coating layered over a component of the device. The reservoirs can have a variety of shapes, sizes and they can be produced by an array of methods. For example, the reservoir can be a monolithic structure located in one or more components of the device or a coating layered over one or more components of the device. Alternatively, the reservoir can be made up of numerous small microcapsules that are, for example, embedded in the material from which the device is fabricated, or in a coating layered over a component of the device. Furthermore, the reservoir can be formed from a coating that includes the bioactive agent diffused throughout, or within a portion, of the coating's three-dimensional structure. The reservoirs can be porous structures that allow the drug to be slowly released from its encapsulation, or the reservoir can include a material that bioerodes following implantation and allows the drug to be released in a controlled fashion.

### 1. Covalently Attached Bioactive Materials

In a preferred embodiment, the biologically active material is covalently bonded to a reactive group located on the sewing ring, the housing component or combinations thereof. The art is replete with methods for preparing derivatized, polymerizable monomers, attaching bioactive materials onto polymeric surfaces and derivatizing bioactive materials and polymers to allow for this attachment *(see,* for example, Hermanson, BIOCONJUGATE TECHNIQUES, Academic Press, 1996, and references therein). Common approaches include the use of coupling agents such as glutaraldehyde, cyanogen bromide, p-benzoquinone, succinic anhydrides, carbodiimides, diisocyanates, ethyl chloroformate, dipyridyl disulfide, epichlorohydrin, azides, among others, which serve as attachment vehicles for coupling reactive groups of biologically active molecules to reactive groups on a monomer or a polymer.

A polymer can be functionalized with reactive groups by, for example, including a moiety bearing a reactive group as an additive to a blend during manufacture of the polymer or polymer precursor. The additive is dispersed throughout the polymer matrix, but does not form an integral part of the polymeric backbone. In this embodiment, the surface of the polymeric material is altered or manipulated by the choice of additive or modifier characteristics. The reactive groups of the additive are used to bind one or more bioactive agents to the polymer.

A useful method of preparing surface-functionalized polymeric materials by this method is set forth in, for example, Caldwell, U.S. Patent No. 5,874,164, issued February 23, 1999. In the Caldwell method, additives or modifiers are combined with the polymeric material during its manufacture. These additives or modifiers include compounds that have reactive sites, compounds that facilitate the controlled release of agents from the polymeric material into the surrounding environment, catalysts, compounds that promote adhesion between the bioactive materials and the polymeric material and compounds that alter the surface chemistry of the polymeric material.

In another embodiment, polymerizable monomers bearing reactive groups are incorporated in the polymerization mixture. The functionalized monomers form part of the polymeric backbone and, preferably, present their reactive groups on the surface of the polymer.

Reactive groups contemplated in the practice of the present invention include functional groups, such as hydroxyl, carboxyl, carboxylic acid, amine groups, and the like, that promote physical and/or chemical interaction with the bioactive material. The particular compound employed as the modifier will depend on the chemical functionality of the biologically active agent and can readily be deduced by one of skill in the art. In the present embodiment, the reactive site binds a bioactive agent by covalent means. It will, however, be apparent to those of skill in the art that these reactive groups can also be used to adhere bioactive agents to the polymer by hydrophobic/hydrophilic, ionic and other non-covalent mechanisms.

In addition to manipulating the composition and structure of the polymer during manufacture, a preferred polymer can also be modified using a surface derivitization technique. There are a number of surface-derivatization techniques appropriate for use in fabricating the prosthetic valves of the present invention (e.g., grafting techniques). These techniques for creating functionalized polymeric surfaces are well known to those skilled in the art. For example, techniques based on ceric ion initiation, ozone exposure, corona discharge, UV irradiation and ionizing radiation (⁶⁰Co, X-rays, high energy electrons, plasma gas discharge) are known and can be used in the practice of the present invention.

Substantially any reactive group that can be reacted with a complementary component on a biologically active material can be incorporated into a polymer and used to covalently attach the biologically active material to the prosthetic valve of the invention or a coating on the valve. In a preferred embodiment, the reactive group is selected from amine-containing groups, hydroxyl groups, carboxyl groups, carbonyl groups, and combinations thereof. In a further preferred embodiment, the reactive group is an amino group.

Aminated polymeric materials useful in practicing the present invention can be readily produced through a number of methods well known in the art. For example, amines may be introduced into a preformed polymer by plasma treatment of materials with ammonia gas as found in Holmes and Schwartz, Composites Science and Technology, 38: 1-21 (1990). Alternatively, amines can be provided by grafting acrylamide to the polymer followed by chemical modification to introduce amine moieties by methods well known to those skilled in the art, e.g., Hofmann rearrangement reaction. A grafted acrylamide-containing polymer may be prepared by radiation grafting as set forth in U.S. Patent. No. 3,826,678 to Hoffman et al. A grafted N-(3-aminopropyl)methacrylamide-containing polymer may be prepared by ceric ion grafting as set forth in U.S. Patent. No. 5,344,455 to Keogh et al., which issued on September 6, 1994. Polyvinylamines or polyalkylimines can also be covalently attached to polyurethane surfaces according to the method taught by U.S. Patent No. 4,521,564 to Solomone et al., which issued on June 5, 1984. Alternatively, for example, aminosilane may be attached to the surface as set forth in U.S. Patent No. 5,053,048 to Pinchuk, which issued on October 1, 1991.

In a preferred embodiment, the prosthetic valve of the invention comprises a polymeric substrate and the reactive chemical functional groups are affixed to the surface of the substrate by plasma fixation. Useful methods for plasma fixation are taught by, for example, Hostettler et al., in U.S. Pat. No. 5,919,570, which issued on July 6, 1999.

In an exemplary embodiment, a coated or uncoated polymeric sewing ring, housing component, valve component, or precursor for subsequent fabrication into these components is exposed to a high frequency plasma with microwaves or, alternatively, to a high frequency plasma combined with magnetic field support to yield the desired reactive surfaces bearing at least a substantial portion of reactant amino groups upon the substrate to be derivatized with the bioactive material.

The functionalized surface can also be prepared by, for example, first submitting a coated or uncoated valve component or component precursor to a chemical oxidation step. This chemical oxidation step is then followed, for example, by exposing the oxidized substrate to one or more plasma gases containing ammonia and/or organic amine(s) which react with the treated surface.

In a preferred embodiment, the gas is selected from the group consisting of ammonia, organic amines, nitrous oxide, nitrogen, and combinations thereof. The nitrogen-containing moieties derived from this gas are preferably selected from amino groups, amido groups, urethane groups, urea groups, and combinations thereof, more preferably primary amino groups, secondary amino groups, and combinations thereof.

In another preferred embodiment, the nitrogen source is an organic amine. Examples of suitable organic amines include, but are not limited to, methylamine, dimethylamine, ethylamine, diethylamine, ethylmethylamine, n-propylamine, allylamine, isopropylamine, n-butylamine, n-butylmethylamine, n-amylamine, n-hexylamine, 2-ethylhexylamine, ethylenediamine, 1,4-butanediamine, 1,6-hexanediamine, cyclohexylamine, n-methylcyclohexylamine, ethyleneimine, and the like.

In a further preferred embodiment, the chemical oxidation step is supplemented with, or replaced by, submitting the polymer to one or more exposures to plasma-gas that contains oxygen. In yet a further preferred embodiment, the oxygen-containing plasma gas further contains argon (Ar) gas to generate free radicals. Immediately after a first-step plasma treatment with oxygen-containing gases, or oxygen/argon plasma gas combinations, the oxidized polymer is preferably functionalized with amine groups. As mentioned above, functionalization with amines can be performed with plasma gases such as ammonia, volatile organic amines, or mixtures thereof.

In the case of very hydrophobic polymer substrates, such as various polyethylenes, the surface is preferably submitted to treatments that render the surface hydrophilic. This treatment is followed by consecutive plasma treatments as described above, to affix reactive functional groups onto the substrates.

In an exemplary embodiment utilizing ammonia and/or organic amines, or mixtures thereof, as the plasma gases, a frequency in the radio frequency (RF) range of from about 13.0 MHz to about 14.0 MHz is used. A generating power of from 0.1 Watts per square centimeter to about 0.5 Watts per square centimeter of surface area of the electrodes of the plasma apparatus is preferably utilized. An exemplary plasma treatment includes evacuating the plasma reaction chamber to a desired base pressure of from about 10 to about 50 mTorr. After the chamber is stabilized to a desired working pressure, ammonia and/or organic amine gases are introduced into the chamber. Preferred flow rates are typically from about 200 to about 650 standard mL per minute. Typical gas pressure ranges from about 0.01 to about 0.5 Torr, and preferably from about 0.2 to about 0.4 Torr. A current having the desired frequency and level of power is supplied by means of electrodes from a suitable external power source. Power output is up to about 500 Watts, preferably from about 100 to about 400 Watts. The plasma treatment can be performed by means of a continuous or batch process.

In the case of batch plasma treatment, a preferred plasma surface treatment system is the PLASMA SCIENCE PS 0350 (HIMONT/PLASMA SCIENCE, Foster City, Calif.).

Optimization procedures for the plasma treatment and the effect of these procedures on the characteristics and the performance of the reactive polymers can be determined by, for example, evaluating the extent of substrate functionalization. Methods for characterizing functionalized polymers are well known in the art.

The result of the above-described exemplary methods is preferably a polymeric surface, which is hydrophilic and which also contains a significant number of primary and/or secondary amino groups. These groups are preferably readily reactive at room temperature with an inherent, or an appended, reactive functional group on the bioactive material.

Once the amine-containing polymeric valve component is prepared, it can be used to covalently bind biologically active molecules having a variety of functional groups including, for example, ketones, aldehydes, activated carboxyl groups (*e*.*g*. activated esters), alkyl halides and the like.

Synthesis of specific biologically active material-polymer conjugates is generally accomplished by: 1) providing a valve component comprising an activated polymer, such as an acrylic acid, and a biologically active agent having a position thereon which will allow a linkage to form; 2) reacting the complementary substituents of the biologically active agent and the polymeric valve component in an inert solvent, such as methylene chloride, chloroform or DMF, in the presence of a coupling reagent, such as 1,3-diisopropylcarbodiimide or any suitable dialkyl carbodiimide (Sigma Chemical), and a base, such as dimethylaminopyridine, diisopropyl ethylamine, pyridine, triethylamine, *etc*. Alternative specific syntheses are readily accessible to those of skill in the art (*see,* for example, Greenwald et al., U.S. Patent No. 5,880,131, issued March 9, 1999.

By way of example, the discussion below is concerned with the attachment of a peptide-based bioactive material to an amine-containing polymeric component of the device of the invention. The choice of a peptide-based biologically active material and an amine-containing polymer is intended to be illustrative of the invention and does not define its scope. It will be apparent to those of skill in the art how to attach a wide range of biologically active agents to polymers comprising amines and other reactive groups.

The conjugates of use in practicing the instant invention, which comprise a peptide, can be synthesized by techniques well known in the medicinal chemistry art. For example, a free amine moiety on a polymeric valve component can be covalently attached to an oligopeptide at the carboxyl terminus such that an amide bond is formed. Similarly, an amide bond may be formed by covalently coupling an amine moiety of an oligopeptide and a carboxyl moiety of a polymeric valve component. For these purposes, a reagent such as 2-(1H-benzotriazol-1-yl)-1,3,3-tetramethyluronium hexafluorophosphate (known as HBTU) and 1-hyroxybenzotriazole hydrate (known as HOBT), dicyclohexylcarbodiimide (DCC), N-ethyl-N-(3-dimethylaminopropyl)-carbodiimide (EDC), diphenylphosphorylazide (DPPA), benzotriazol-1-yl-oxy-tris-(dimethylamino)phosphonium hexafluorophosphate (BOP) and the like, in combination, or singularly, can be utilized.

Furthermore, the instant conjugate can be formed by a non-peptidyl bond between a peptide and a polymeric valve component. For example, a peptide can be attached to a polymeric valve component through a carboxyl terminus of an oligopeptide via a hydroxyl moiety on a polymeric valve component, thereby forming an ester linkage. For this purpose, a reagent such as a combination of HBTU and HOBT, a combination of BOP and imidazole, a combination of DCC and DMAP, and the like can be utilized.

The instant conjugate can also be formed by attaching the oligopeptide to the polymeric valve component via a linker unit. Such linker units include, for example, a biscarbonyl alkyl diradical whereby an amine moiety on the polymeric valve component is connected with the linker unit to form an amide bond and the amino terminus of the oligopeptide is connected with the other end of the linker unit also forming an amide bond. Conversely, a diaminoalkyl diradical linker unit, whereby a carbonyl moiety on the polymeric valve component is covalently attached to one of the amines of the linker unit while the other amine of the linker unit is covalently attached to the C-terminus of the oligopeptide, can also be utilized. Other such linker units which are stable to the physiological environment, are also envisioned.

In addition to linkers that are stable *in vivo*, linkers that are designed to be cleaved to release the biolog cally active agent from the polymer are useful in the prosthetic devices of the present invention. Many such linker arms are accessible to those of skill in the art. Common cleavable linker arms include, for example, specific protease cleavage sequences, disulfides, esters and the like. Many appropriate cleavable cross-linking agents are commercially available from companies, such as Pierce (Rockford, IL), or can be prepared by art-recognized methods.

Any of the bioactive agents from the various classes of bioactive agents set forth above can be tethered to a polymer by the methods described herein. In a particularly preferred embodiment, the biologically active material is a taxane. For purposes of the present invention, the term "taxane" includes all compounds within the taxane family of terpenes. Thus, taxol (paclitaxel), 3'-substituted tert-butoxy-carbonyl-amine derivatives (taxoteres) and the like as well as other analogs available from, for example, Sigma Chemical (St. Louis, MO.) and/or Bristol Meyers Squibb are within the scope of the present invention.

Generally, it is preferred that a taxane having the 2' position available for substitution is reacted with a suitably activated polymer such as a polymeric carboxylic acid under conditions sufficient to cause the formation of a 2' ester linkage between the two substituents.

One skilled in the art understands that in the synthesis of compounds useful in practicing the present invention, one may need to protect various reactive functionalities on the starting compounds and intermediates while a desired reaction is carried out on other portions of the molecule. After the desired reactions are complete, or at any desired time, normally such protecting groups will be removed by, for example, hydrolytic or hydrogenolytic means. Such protection and deprotection steps are conventional in organic chemistry. One skilled in the art is referred to PROTECTIVE GROUPS IN ORGANIC CHEMISTRY, McOmie, ed., Plenum Press, NY, N.Y. (1973); and, PROTECTIVE GROUPS IN ORGANIC SYNTHESIS, Greene, ed., John Wiley & Sons, NY (1981) for the teaching of protective groups which may be useful in the preparation of compounds of the present invention.

In another preferred embodiment, the bioactive agent is covalently bound to a material that coats one or more components of the prosthetic valve of the invention. The discussion above regarding functionalizing of polymers is generally relevant to embodiments in which the bioactive agent is covalently attached to one or more components of a species coating the prosthetic valve. The agent can be attached to the coating in a manner that is either reversible (e.g., through a cleavable linker) or irreversible. Exemplary coatings to which the bioactive agent can be attached include, for example, synthetic polymers (e.g., poly(urethanes), poly(acrylamides), *etc*.), "natural" polymers (e.g., polylactic acid, polyglycolic acid, polyamino acid, *etc.)* hydrogels, thermoreversible gels, pluronics, fibrin sealants, and the like. Methods for preparing monomers functionalized with a particular bioactive agent, and for functionalizing polymeric materials with a bioactive agent are well known and accessible to those of skill in the art.

In yet another preferred embodiment, one or more bioactive agent is bound to both the polymer from which a component of the prosthetic valve is constructed and a coating layered over one or more components of the prosthetic valve. The characteristics of bioactive agents bound to a valve component and a those of a bioactive agent bound to a coating component set forth above are equally applicable to this embodiment.

### 2. Reversibly Associated Bioactive Materials

For the purpose of illustration, the following discussion is primarily focused on bioactive agents that are reversibly associated with a coating on a prosthetic valve of the invention. This focus is not intended to be limiting and it will be apparent to those of skill that the general principles set forth are equally applicable to embodiments in which the bioactive agent is reversibly associated with a component of the prosthetic valve itself or reversibly associated with both a component of the valve itself and a coating on the valve.

Generally, if it is desired that the biologically active agent remain active at the surface of a component of the prosthetic valve for a long period of time, it is preferable to covalently attach the biologically active molecule to the device itself. In contrast, if it is desired that the biologically active agent diffuse out of the prosthetic valve, the agent should be reversibly adhered to the prosthetic or to a coating on the prosthetic. Alternatively, the agent cane be bound to the prosthetic or a coating on the prosthetic by means of a cleavable linker. The reversibly adhered agents can be exposed on the prosthetic surface, they can be covered with a coating, such as a bioerodable polymer, or they can be encapsulated in, for example, a microparticle embedded in a coating or in a component of the prosthetic valve itself.

As used herein, the term "reversibly associated" refers to species that are associated with the device of the invention in a non-covalent manner or via a linkage that is cleaved following the implantation of the device. Mechanisms underlying non-covalent forms of reversible association include, for example, hydrophilic/hydrophobic, ionic, van der Waals, and like interactions.

Methods for altering the hydrophobicity/hydrophilicity of monomers, polymers and fabrics are well known in the art. For example, various polyorganosiloxane compositions are taught in the prior art that can be used for making coatings imparting hydrophobicity to fabrics. Typical of such teachings is the process described in Takamizawa, et al., U.S. Patent No. 4,370,365, issued January 25, 1983, which describes a hydrophobizing agent comprising, in addition to an organohydrogenpolysiloxane, either one or a combination of linear organopolysiloxanes containing alkene groups, and a resinous organopolysiloxane containing tetrafunctional and monofunctional siloxane units. The resultant mixture is catalyzed for curing and dispersed into an aqueous emulsion. The fabric is dipped in the emulsion and heated, producing a hydrophobic fabric.

In a exemplary embodiment, the surface character of the polymeric material is altered or manipulated by including certain additives or modifiers in the polymeric material during its manufacture. A method of preparing surface-functionalized polymeric materials by this method is set forth in, for example, Caldwell, *supra*. In the Caldwell method, additives or modifiers are combined with the polymeric material during its manufacture. These additives or modifiers include compounds that have affinity sites, compounds that facilitate the controlled release of agents from the polymeric material into the surrounding environment, catalysts, compounds that promote adhesion between the bioactive materials and the polymeric material and compounds that alter the surface chemistry of the polymeric material.

As used herein, the term "affinity site" refers to a site on the polymer that interacts, in a non-covalent manner, with a complementary site on a biologically active agent or a complementary site on a target tissue, organ, cell, *etc*.

Affinity sites contemplated in the practice of the present invention include such functional groups as hydroxyl, carboxyl, carboxylic acid, amine groups, hydrophobic groups, inclusion moieties (*e*.*g*., cyclodextrin, complexing agents), biomolecules (e.g., antibodies, haptens, saccharides, peptides) and the like, that promote physical and/or chemical interaction with the bioactive material. In the present embodiment, the affinity site interacts with a bioactive agent by non-covalent means. The particular compound employed as the modifier will depend on the chemical functionality of the biologically active agent and appropriate functional groups for a particular purpose can readily be deduced by one of skill in the art.

In an exemplary embodiment, the polymeric material is a fabric and it is treated with a biologically active agent by impregnating or saturating the fabric with a solution of the bioactive agent. The impregnation step can be carried out under pressure to further drive the bioactive material into the fabric matrix. The pressure can also be used to drive bioactive agents to various selected positions within the polymeric material. The extent and rate of migration can be controlled by controlling the viscosity and thickness of the solution containing the bioactive molecule. The application of pressure can be repeated until the bioactive material is finally moved and/or fixed into the preselected position.

The amount of biologically active material used in this process can vary depending on the activity of the material and the tenaciousness with which it adheres to the polymeric material. After the impregnation step, the fabric can be cured by, for example, heat, radiation or both to further adhere the biologically active compound to the polymeric material.

In another preferred embodiment, the biologically active material interacts with a surfactant that adheres to the surface of the polymeric material. Presently preferred surfactants are selected from benzalkonium halides and sterylalkonium halides. Other surfactants suitable for use in the present invention are known to those of skill in the art.

In a still further preferred embodiment, the bioactive material interacting with, and adhering to, the polymeric material is a taxane, a taxane derivative or a combination thereof.

### E. Coated Prosthetic Devices

In another preferred embodiment, the prosthetic valve of the invention further comprises a coating layer over a polymeric component of the prosthetic valve. In a still further preferred embodiment, the sewing ring, the housing component, the valve components within the housing, and combinations thereof are at least partially covered with a coating for release of at least one biologically active material. Other preferred prosthetic valves comprise a reservoir component formed by, or integral to, the coating. The reservoir contains the biologically active material and controls its release properties.

The coating can take a number of forms. For example, useful coatings can be in the form of gels (e.g. hydrogel, thermoreversible gel) foams, suspensions, microcapsules, solid polymeric materials and fibrous or porous structures. The coating can be multilayered with one or more of the layers including a biologically active material. Moreover, the coating can be layered over a valve component that is impregnated with a biologically active agent, or that has a biologically active agent immobilized thereon. Alternatively, the bioactive agent can be dispersed in one or more components or regions of the coating, or within a microparticle, which is itself contained within the coating. Many materials that are appropriate for use as coatings in the present prosthetic valves are known in the art and both natural and synthetic coatings are useful in practicing the present invention.

### 1. Selection of Coating Materials

Suitable polymers that can be used as coatings in the present invention include, but are not limited to, water soluble and water insoluble, biodegradable and nonbiodegradable polymers. The coatings of use in the present invention are preferably biodegradable, or more preferably bioerodable. The coatings are preferably sufficiently porous, or capable of becoming sufficiently porous, to permit efflux of the biologically active molecules from the coating. The coatings are also preferably sufficiently non-inflammatory and are biocompatible so that inflammatory responses do not prevent the delivery of the biologically active molecules to the tissue. It is advantageous if the coating also provides at least partial protection of the biologically active molecules from the adverse effects of proteases, hydrolases, nucleases and other relevant degradative species. In addition, it is advantageous if controlled, sustained delivery can be obtained using the polymeric carriers.

Many polymers can be utilized to form the coating. A coating can be, for example, a gel, such as a hydrogel, organogel or thermoreversible gel. Other useful polymer types include, but are not limited to, thermoplastics and films. Moreover, the coating can comprise a homopolymer, copolymer or a blend of these polymer types. The coating can also include a drug-loaded microparticle dispersed within a component of the coating, which serves as a dispersant for the microparticles. Microparticles include, for example, microspheres, microcapsules and liposomes.

The coating matrix can serve to immobilize the microparticles at a particular site, enhancing targeted delivery of the encapsulated biologically active molecules. Rapidly bioerodible polymers such as polylactide-co-glycolide, polyanhydrides, and polyorthoesters, whose carboxylic groups are exposed on the surface are useful in the coatings of use in the invention. In addition, polymers containing labile bonds, such as polyesters, are well known for their hydrolytic reactivity. The hydrolytic degradation rates of the coatings can generally be altered by simple changes in the polymer backbone.

The coating can be made up of natural and/or synthetic polymeric materials. Representative natural polymers of use as coatings in the present invention include, but are not limited to, proteins, such as zein, modified zein, casein, gelatin, gluten, serum albumin, or collagen, and polysaccharides, such as cellulose, dextrans, and polyhyaluronic acid. Also of use in practicing the present invention are materials, such as collagen and gelatin, which have been widely used on implantable devices, such as textile grafts (*see,* for example, Hoffman, et al., U.S. Patent Nos. 4,842,575, which issued on June 27, 1989 and 5,034,265, which issued on July 23, 1991), but which have not been utilized as components of adherent coatings for periadventitial delivery of bioactive agents, such as those preventing or retarding the development if intimal hyperpalsia. Hydrogel or sol-gel mixtures of polysaccharides are also known. Furthermore, fibrin, an insoluble protein formed during the blood clotting process, has also been used as a sealant for porous implantable devices (*see*, for example, Sawhey et al., U.S. Patent No. 5,900,245, issued May 4, 1999). Useful fibrin sealant compositions are disclosed in, for example, Edwardson et al., U.S. Patent No. 5,770,194, which issued on June 23, 1998 and U.S. Patent No. 5739288, which issued on April 14, 1998. These and other naturally based agents, alone or in combination, can be used as a coating in practicing the present invention.

Representative synthetic polymers include, but are not limited to, polyphosphazines, poly(vinyl alcohols), polyamides, polycarbonates, polyalkylenes, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate) polyethylene, polypropylene, poly(ethylene glycol), poly(ethylene oxide), poly (ethylene terephthalate), poly(vinyl acetate), polyvinyl chloride, polystyrene, polyvinyl pyrrolidone, pluronics and polyvinylphenol and copolymers thereof.

Synthetically modified natural polymers include, but are not limited to, alkyl celluloses, hydroxyalkyl celluloses, cellulose ethers, cellulose esters, and nitrocelluloses. Particularly preferred members of the broad classes of synthetically modified natural polymers include, but are not limited to, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, and polymers of acrylic and methacrylic esters and alginic acid.

These and the other polymers discussed herein can be readily obtained from commercial sources such as Sigma Chemical Co. (St. Louis, MO.), Polysciences (Warrenton, PA.), Aldrich (Milwaukee, WI.), Fluka (Ronkonkoma, NY), and BioRad (Richmond, CA), or else synthesized from monomers obtained from these suppliers using standard techniques.

### 2. Biodegradable and Bioresorbable Coating Materials

Coating compositions preferably have intrinsic and controllable biodegradability, so that they persist for about a week to about six months. The coatings are also preferably biocompatible, non-toxic, contain no significantly toxic monomers and degrade into non-toxic components. Moreover, preferred coatings are chemically compatible with the substances to be delivered, and tend not to denature the active substance. Still further preferred coatings are, or become, sufficiently porous to allow the incorporation of biologically active molecules and their subsequent liberation from the coating by diffusion, erosion or a combination thereof. The coatings should also remain at the site of application by adherence or by geometric factors, such as by being formed in place or softened and subsequently molded or formed into microparticles which are trapped at a desired location. Types of monomers, macromers, and polymers that can be used are described in more detail below.

Representative biodegradable polyme-s include, but are not limited to, polylactides, polyglycolides and copolymers thereof, poly(ethylene terephthalate), poly(butyric acid), poly(valeric acid), poly(lactide-cc-caprolactone), poly(lactide-co-glycolide), polyanhydrides, polyorthoesters, blends and copolymers thereof. Of particular use are compositions that form gels, such as those including collagen (*e*.*g*., lightly crosslinked), pluronics, gelatin and the like.

Still further preferred coatings are water-insoluble materials that comprise within at least a portion of their structure, a bioresorbable molecule. An example of such a coating is one that includes a water-insoluble copolymer which has a bioresorbable region, a hydrophilic region and a plurality of crosslinkable functional groups per polymer chain.

For purposes of the present invention, "water-insoluble" is intended to mean that the copolymers of the present invention are substantially insoluble in water or water-containing environments. Thus, although certain regions or segments of the copolymer may be hydrophilic or even water-soluble, the copolymer molecule, as a whole, does not by any substantial measure dissolve in water or water-containing environments.

For purposes of the present invention, the term "bioresorbable" means that this region is capable of being metabolized or broken down and resorbed and/or eliminated through normal excretory routes by the body. Such metabolites or break-down products should be substantially non-toxic to the body.

The bioresorbable region is preferably hydrophobic. In another embodiment, however, the bioresorbable region may be designed to be hydrophilic so long as the copolymer composition as a whole is not rendered water-soluble. Thus, the bioresorbable region is designed based on the preference that the copolymer, as a whole, remains water-insoluble. Accordingly, the relative properties, i.e., the kinds of functional groups contained by, and the relative proportions of the bioresorbable region, and the hydrophilic region are selected to ensure that useful bioresorbable compositions remain water-insoluble.

Exemplary resorbable coatings include, for example, synthetically produced resorbable block copolymers of poly(α-hydroxy-carboxylic acid)/poly(oxyalkylene, *(see,* Cohn et al., U.S. Patent No. 4,826,945). These copolymers are not crosslinked and are water-soluble so that the body can excrete the degraded block copolymer compositions. *See*, Younes et al., J Biomed. Mater. Res. 21: 1301-1316 (1987); and Cohn et al., J Biomed. Mater. Res. 22: 993-1009 (1988).

Presently preferred bioresorbable polymers include one or more components selected from pc ly(esters), poly(hydroxy acids), poly(lactones), poly(amides), poly(ester-amides), poly (amino acids), poly(anhydrides), poly(orthoesters), poly(carbonates), poly(phosphazines), poly(phosphoesters), poly(thioesters), polysaccharides and mixtures thereof. More preferably still, the biosresorbable polymer includes a poly(hydroxy) acid component. Of the poly(hydroxy) acids, polylactic acid, polyglycolic acid, polycaproic acid, polybutyric acid, polyvaleric acid, fibrin and copolymers and mixtures thereof are preferred.

In addition to forming fragments that are absorbed *in vivo* ("bioresorbed"), preferred polymeric coatings for use in the prosthetic valves of the invention can also form an excretable and/or metabolizable fragment.

Higher order copolymers can also be used as coatings in the prosthetic valves of the present invention. For example, Casey et al., U.S. Patent No. 4,438,253, which issued on March 20, 1984, discloses tri-block copolymers produced from the transesterification of poly(glycolic acid) and an hydroxyl-ended poly(alkylene glycol). Such compositions are disclosed for use as resorbable monofilament sutures. The flexibility of such compositions is controlled by the incorporation of an aromatic orthocarbonate, such as tetra-p-tolyl orthocarbonate into the copolymer structure.

Other coatings based on lactic and/or glycolic acids can also be utilized. For example, Spinu, U.S. Patent No. 5,202,413, which issued on April 13, 1993, discloses biodegradable multi-block copolymers having sequentially ordered blocks of polylactide and/or polyglycolide produced by ring-opening polymerization of lactide and/or glycolide onto either an oligomeric diol or a diamine residue followed by chain extension with a difunctional compound, such as, a diisocyanate, diacylchloride or dichlorosilane.

The monomers, polymers and copolymers of use in the present invention preferably form a stable aqueous emulsion, and more preferably a flowable liquid. The relative proportions or ratios of the bioresorbable and hydrophilic regions, respectively are preferably selected to render the block copolymer composition water-insoluble. Furthermore, these compositions are preferably sufficiently hydrophilic to form a hydrogel in aqueous environments when crosslinked.

The specific ratio of the two regions of the block copolymer composition for use as coatings in the present invention will vary depending upon the intended application and will be affected by the desired physical properties of the implantable prosthetic valve, the site of implantation, as well as other factors. For example, the composition of the present invention will preferably remain substantially water-insoluble when the ratio of the water-insoluble region to the hydrophilic region is from about 10:1 to about 1:1, on a percent by weight basis.

Preferred bioresorbable regions of coatings useful in the present invention can be designed to be hydrolytically and/or enzymatically cleavable. For purposes of the present invention, "hydrolytically cleavable" refers to the susceptibility of the copolymer, especially the bioresorbable region, to hydrolysis in water or a water-containing environment. Similarly, "enzymatically cleavable" as used herein refers to the susceptibility of the copolymer, especially the bioresorbable region, to cleavage by endogenous or exogenous enzymes.

As set forth above, the preferred composition also includes a hydrophilic region. Although the present composition contains a hydrophilic region, in preferred coatings, this region is designed and/or selected so that the composition as a whole, remains substantially water-insoluble.

When placed within the body, the hydrophilic region can be processed into excretable and/or metabolizable fragments. Thus, the hydrophilic region can include, for example, polyethers, polyalkylene oxides, polyols, poly(vinyl pyrrolidine), poly(vinyl alcohol), poly(alkyl oxazolines), polysaccharides, carbohydrates, peptides, proteins and copolymers and mixtures thereof. Furthermore, the hydrophilic region can also be, for example, a poly(alkylene) oxide. Such poly(alkylene) oxides can include, for example, poly(ethylene) oxide, poly(propylene) oxide and mixtures and copolymers thereof.

Concerning the disposition of the biologically active agent in the coating, substantially any combination of bioactive compound and coating that is of use in achieving the object of the present invention is contemplated by this invention. In a preferred embodiment, the bioactive material is dispersed in a resorbable coating that imparts controlled release properties to the biologically active agent. The controlled release properties can result from, for example, a resorbable polymer that is cross-linked with a degradable cross-linking agent. Alternatively, the controlled release properties can arise from a resorbable polymer that incorporates the biologically active material in a network of pores formed during the cross-linking process or gelling. In another embodiment, the drug is loaded into microspheres, which are themselves biodegradable and the microspheres are embedded in the coating. Many other appropriate drug/coating formats will be apparent to those of skill in the art.

In another preferred embodiment, an underlying polymeric component of a coating of use in the invention is first impregnated with the biologically active material and a resorbable polymer is layered onto the underlying component. In this embodiment, the impregnated component serves as a reservoir for the bioactive material, which can diffuse out through pores in a resorbable polymer network, through voids in a polymer network created as a resorbable polymer degrades *in vivo*, or through a layer of a gel-like coating. Other controlled release formats utilizing a polymeric substrate, a bioactive agent and a coating will be apparent to those of skill in the art.

### 3. Hydrogel-based Coatings

Also contemplated for use in the practice of the present invention as a coating component are hydrogels. Hydrogels are polymeric materials that are capable of absorbing relatively large quantities of water. Examples of hydrogel forming compounds include polyacrylic acids, sodium carboxymethylcellulose, polyvinyl alcohol, polyvinyl pyrrolidine, gelatin, carrageenan and other polysaccharides, hydroxyethylenemethacrylic acid (HEMA), as well as derivatives thereof, and the like. Hydrogels can be produced that are stable, biodegradable and bioresorbable. Moreover, hydrogel compositions can include subunits that exhibit one or more of these properties.

Biocompatible hydrogel compositions whose integrity can be controlled through crosslinking are known and are presently preferred for use in the prosthetic valves of the invention. For example, Hubbell et al., U.S. Patent Nos. 5,410,016, which issued on April 25, 1995 and 5,529,914, which issued on June 25, 1996, disclose water-soluble systems, which are crosslinked block copolymers having a water-soluble central block segment sandwiched between two hydrolytically labile extensions. Such copolymers are further end-capped with photopolymerizable acrylate functionalities. When crosslinked, these systems become hydrogels. The water soluble central block of such copolymers can include poly(ethylene glycol); whereas, the hydrolytically labile extensions can be a poly(α-hydroxy acid), such as polyglycolic acid or polylactic acid. *See*, Sawhney et al., Macromolecules 26: 581-587 (1993).

In a preferred embodiment, the bioactive material is dispersed in a hydrogel that is cross-linked to a degree sufficient to impart controlled release properties to the biologically active agent. The controlled release properties can result from, for example, a hydrogel that is cross-linked with a degradable cross-linking agent. Alternatively, the controlled release properties can arise from a hydrogel that incorporates the biologically active material in a network of pores formed during the cross-linking process.

In another preferred embodiment, the gel is a thermoreversible gel. Thermoreversible gels including components, such as pluronics, collagen, gelatin, hyalouronic acid, polysaccharides, polyurethane hydrogel, polyurethane-urea hydrogel and combinations thereof are presently preferred.

In yet another preferred embodiment, the polymeric material of a component of the prosthetic valve is first impregnated with the biologically active material and the hydrogel is layered onto the impregnated valve component. In this embodiment, the impregnated device component serves as a reservoir for the bioactive material or agent, which can diffuse out through pores in the hydrogel network or, alternatively, can diffuse out through voids in the network created as the hydrogel degrades *in vivo* (*see*, for example, Ding et al., U.S. Patent No. 5,879,697, issued March 9, 1999; and Ding et al., U.S. Patent No. 5,837,313, issued November 17, 1998). Other controlled release formats utilizing a polymeric substrate, a bioactive agent and a hydrogel will be apparent to those of skill in the art.

As set forth above, useful coatings of the present invention can also include a plurality of crosslinkable functional groups. Any crosslinkable functional group can be incorporated into these compositions so long as it permits or facilitates the formation of a hydrogel. Preferably, the crosslinkable functional groups of the present invention are olefinically unsaturated groups. Suitable olefinically unsaturated functional groups include without limitation, for example, acrylates, methacrylates, butenates, maleates, allyl ethers, allyl thioesters and N-allyl carbamates. Preferably, the crosslinking agent is a free radical initiator, such as for example, 2,2'-azobis (N,N'dimethyleneisobutyramidine) dihydrochloride.

The crosslinkable functional groups can be present at any point along the polymer chain of the present composition so long as their location does not interfere with the intended function thereof. Furthermore, the crosslinkable functional groups can be present in the polymer chain of the present invention in numbers greater than two, so long as the intended function of the present composition is not compromised.

An example of a coating having the above-recited characteristics is found in, for example, Loomis, U.S. Patent No. 5,854,382, issued December 29, 1998. This coating is exemplary of the types of coatings that can be used in the prosthetic valve of the invention.

Also contemplated by the present invention is the use of coatings that are capable of promoting the release of an agent from the coated device. For example, in a preferred embodiment, the bioactive material is dispersed throughout the hydrogel. As the hydrogel degrades by hydrolysis or enzymatic action, the bioactive material is released. Alternatively, the coating may promote the release of a biologically active material by forming pores once the resulting article is placed in a particular environment (*e*.*g*., *in vivo*). In a preferred embodiment, these pores communicate with a reservoir containing the bioactive material. Other such coating components that promote the release of an agent from materials are known to those of skill in the art.

### F. Microencapsulation of Bioactive Material

In the present invention, the biologically active material is incorporated into a polymeric component by encapsulation in a microcapsule. The microcapsule is preferably fabricated from a material different from that of the polymeric component and the bulk of the coating matrix.

Preferred microcapsules are those which are fabricated from a material that undergoes erosion in the host, or those which are fabricated such that they allow the bioactive agent to diffuse out of the microcapsule. Such microcapsules can be used to provide for the controlled release of the encapsulated biologically active material from the microcapsules.

Numerous methods are known for preparing microparticles of any particular size range. In the various prosthetic valves of the present invention, the microparticle sizes may range from about 0.2 micron up to about 100 microns. Synthetic methods for gel microparticles, or for microparticles from molten materials, are known, and include polymerization in emulsion, in sprayed drops, and in separated phases. For solid materials or preformed gels, known methods include wet or dry milling or grinding, pulverization, size separation by air jet, sieve, and the like.

Microparticles can be fabricated from different polymers using a variety of different methods known to those skilled in the art. Exemplary methods include those set forth below detailing the preparation of polylactic acid and other microparticles.

Polylactic acid microparticles are preferably fabricated using one of three methods: solvent evaporation, as described by Mathiowitz, et al., J. Scanning Microscopy 4:329 (1990); Beck, et al., Fertil. Steril. 31: 545 (1979); and Benita, et al., J. Pharm. Sci. 73: 1721 (1984); hot-melt microencapsulation, as described by Mathiowitz, et al., Reactive Polymers 6: 275 (1987); and spray drying. Exemplary methods for preparing microencapsulated bioactive materials useful in practicing the present invention are set forth below.

### 1. Solvent Evaporation

In this method, the microcapsule polymer is dissolved in a volatile organic solvent, such as methylene chloride. The drug (either soluble or dispersed as fine particles) is added to the solution, and the mixture is suspended in an aqueous solution that contains a surface active agent such as poly(vinyl alcohol). The resulting emulsion is stirred until most of the organic solvent has evaporated, leaving solid microparticles. The solution is loaded with a drug and suspended in vigorously stirred distilled water containing poly(vinyl alcohol) (Sigma). After a period of stirring, the organic solvent evaporates from the polymer, and the resulting microparticles are washed with water and dried overnight in a lyophilizer. Microparticles with different sizes (1-1000 microns) and morphologies can be obtained by this method. This method is useful for relatively stable polymers like polyesters and polystyrene. Labile polymers such as polyanhydrides, may degrade during the fabrication process due to the presence of water. For these polymers, the following two methods, which are performed in completely anhydrous organic solvents, are preferably used.

### 2. Hot Melt Microencapsulation

In this method, the polymer is first melted and then mixed with the solid particles of biologically active material that have preferably been sieved to less than 50 microns. The mixture is suspended in a non-miscible solvent (like silicon oil) and, with continuous stirring, heated to about 5 °C above the melting point of the polymer. Once the emulsion is stabilized, it is cooled until the polymer particles solidify. The resulting microparticles are washed by decantation with a solvent such as petroleum ether to give a free-flowing powder. Microparticles with sizes ranging from about 1 to about 1000 microns are obtained with this method. The external surfaces of capsules prepared with this technique are usually smooth and dense. This procedure is preferably used to prepare microparticles made of polyesters and polyanhydrides.

### 3. Solvent Removal

This technique is preferred for polyanhydrides. In this method, the biologically active material is dispersed or dissolved in a solution of the selected polymer in a volatile organic solvent like methylene chloride. This mixture is suspended by stirring in an organic oil (such as silicon oil) to form an emulsion. Unlike solvent evaporation, this method can be used to make microparticles from polymers with high melting points and different molecular weights. Microparticles that range from about 1 to about 300 microns can be obtained by this procedure. The external morphology of spheres produced with this technique is highly dependent on the type of polymer used.

### 4. Spray-Drying

In this method, the polymer is dissolved in methylene chloride. A known amount of the active drug is suspended (insoluble drugs) or co-dissolved (soluble drugs) in the polymer solution. The solution or the dispersion is then spray-dried. Microparticles ranging between about 1 to about 10 microns are obtained with a morphology which depends on the type of polymer used.

### 5. Hydrogel Microparticles

In a preferred embodiment, the bioactive material is encapsulated in microcapsules that comprise a sodium alginate envelope.

Microparticles made of gel-type polymers, such as alginate, are preferably produced through traditional ionic gelation techniques. The polymers are first dissolved in an aqueous solution, mixed with barium sulfate or some bioactive agent, and then extruded through a microdroplet forming device, which in some instances employs a flow of nitrogen gas to break off the droplet. A slowly stirred (approximately 100-170 RPM) ionic hardening bath is positioned below the extruding device to catch the forming microdroplets. The microparticles are left to incubate in the bath for about twenty to thirty minutes in order to allow sufficient time for gelation to occur. Microparticle size is controlled by using various size extruders or varying either the nitrogen gas or polymer solution flow rates.

### 6. Liposomes

Liposomes are commercially available from a variety of suppliers. Alternatively, liposomes can be prepared according to methods known to those skilled in the art, for example, as described in Eppstein et al., U.S. Patent No. 4,522,811, which issued on June 11, 1985. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its pharmaceutically acceptable salt is then introduced into the container. The container is then swirled by hand to free lipid material from the sides of the container and to disperse lipid aggregates, thereby forming the liposomal suspension.

The above-recited microparticles and methods of preparing the microparticles are offered by way of example and they are not intended to define the scope of microparticles of use in the present invention. It will be apparent to those of skill in the art that an array of microparticles, fabricated by different methods, are of use in the present invention.

### G. Bioactive Agent Release Rates

In another preferred embodiment, the prosthetic valve of the invention includes two or more populations of bioactive agents. The populations are distinguished by, for example, having different rates of release from the prosthetic valve of the invention. Two or more different rates of release can be obtained by, for example, incorporating one agent population into the bulk coating and the other agent population into microcapsules embedded in the bulk coating. In another exemplary embodiment, the two agents are encapsulated in microspheres having distinct release properties. For example, the first agent is encapsulated in a liposome and the second agent is encapsulated in an alginate microsphere.

Other characteristics of the populations in addition to their release rates can be varied as well. For example, the two populations can consist of the same, or different agents. Moreover, the concentrations of the two populations can differ from each other. For example, in certain applications it is desirable to have one agent released rapidly (e.g., an antibiotic) at a first concentration, while a second agent is released more slowly at a second concentration (e.g., an inhibitor of tissue overgrowth). Many other such permutations of agent types, agent concentrations and agent release rates will be readily apparent to those of skill in the art.

### H. Characterization

Characterization of the bioactive agent, the coatings and the combination of the bioactive agent and the coating can be performed at different loadings of bioactive material to investigate coating and encapsulation properties and morphological characteristics of the coatings and microparticles. Microparticle size can be measured by quasi-elastic light scattering (QELS), size-exclusion chromatography (SEC) and the like. Drug loading can be measured by dissolving the coating or the microparticles into an appropriate solvent and assaying the amount of biologically active molecules using one or more art-recognized techniques. Useful techniques include, for example, spectroscopy *(e.g.,* IR, NMR, UV/Vis, fluorescence, *etc.),* mass spectrometry, elemental analysis, HPLC, HPLC coupled with one or more spectroscopic modalities, and other appropriate means.

Numerous *in vivo* and *in vitro* assays can be used to assess the extent of tissue growth on a prosthetic valve incorporating one or more bioactive agents. Useful *in vivo* assays include, but are not limited to, implantation assays. In one such assay, a prosthetic heart valve of the invention is implanted into a recipient (*e*.*g*., sheep, dog, cow, primate, *etc*.). The performance of the valve is monitored for degeneration over time. At the end of a set period of time, the animal is sacrificed and the valve is examined for the presence and/or extent of pannus growth.

Useful *in vitro* assays include, for example, cell adhesion studies using isolated cells and monitoring their ability to attach to the bioactive agent treated material of the prosthetic valve. Other in vitro assays include, but are not limited to, cell migration using organ culture or confluent cell layers either with or without a support matrix. Other assays useful in conjunction with monitoring the growth of pannus onto the prosthetic valve of the invention will be apparent to those of skill in the art.

## Claims

1. A prosthetic heart valve resistant to tissue overgrowth following imputation of said prosthetic heart valve into a host, said heart valve comprising a sewing ring, and a housing component enclosing a valve component, wherein an antimicrotubule agent, in an amount sufficient to prevent tissue overgrowth, is incorporated into the sewing ting.

2. A heart valve according to Claim 1, wherein said antimicrotubule agent is incorporated into a reservoir located on the prosthetic heart valve.

3. A heart valve according to Claim 1 or Claim 2, wherein said antimicrotubule agent is encapsulated in a microcapsule to form a microencapsulated antimicrotubule agent.

4. A heart valve according to Claim 1, wherein said antimicrotubule agent is encapsulated in a microcapsule to form a microencapsulated antimicrotubule agent and said microencapsulated antimicrotubule agent is embedded in the material from which the prosthetic heart valve is fabricated.

5. A heart valve according to any preceding claim, wherein said sewing ring comprises a polymeric material.

6. A heart valve according to Claim 5, wherein said polymeric material comprises a member selected from plastics, rubbers and combinations thereof.

7. A heart valve according to Claim 5, wherein said polymeric material is a fabric.

8. A heart valve according to Claim 7, wherein said fabric comprises a material that is a member selected from thermoplastic polyurethanes TPUs, nylons, polypropylene, polytetrafluoroethylene, polyesters, nylon polymers, block copolymers of a polyether polymer and a polyester polymer, and block copolymers of a polyether polyol and one selected from the group consisting of polyamides, polyimides, polyolefins, synthetic hydrocarbon elastomers, and natural rubber.

9. A heart valve according to Claim 8, wherein said polyester is polyethylene terephthalate (PET).

10. A heart valve according to Claim 8, wherein said nylon is a member selected from pylon-11, nylone-12 and combinations thereof.

11. A heart valve according to Claim 8, wherein said polyolefin is a member selected from polyethylenes (PE) and polypropylenes (PP).

12. A heart valve according to any one of Claims 7 to 11, wherein said fabric is a member selected from a weft knit with a velour, a weft knit without a velour, a warp knit with a velour, a warp knit without a velour, a weave structure with a velour, a weave structure without a velour and combinations thereof.

13. A heart valve according to any one of Claims 7 to 12, wherein said fabric comprises a combination yarn comprising at least two polymeric components.

14. A heart valve according to Claim 13, wherein said combination yarn comprises polyester wrapped with polypropylene yarn.

15. A heart valve according to any preceding claim, wherein said antimicrotubule agent is a member selected from taxane, taxane derivatives and combinations thereof.

16. A heart valve according to any preceding claim, further comprising a coating layer.

17. A heart valve according to any one of Claims 3 to 16, wherein said coating is layered over said microcapsule.

18. A heart valve according to Claim 16 or Claim 17, wherein said coating is a member selected from bioerodable coatings, hydrogel coatings, thermoreversible coatings, bioresorbable coatings and combinations thereof.

19. A heart valve according to any one of Claims 3 to 18, wherein said microcapsule is fabricated from a material that undergoes erosion in said host, thereby providing for controlled release of said encapsulated antimicrotubule agent from said microcapsules.

20. A heart valve according to Claim 19, wherein said microcapsule comprise a sodium alginate envelope.

## Patentansprüche

1. Künstliche Herzklappe, die, nach Implantation der besagten künstlichen Herzklappe in einen Wirt, gegen Gewebewachstum beständig ist, wobei besagte Herzklappe einen Nähring umfasst und eine Gehäusekomponente eine Klappenkomponente umgibt, wobei ein Anti-Mikrotubulusmittel, in einer zur Verhinderung von Gewebewachstum ausreichenden Menge, in den Nähring inkorporiert ist.

2. Herzklappe nach Anspruch 1, wobei besagtes Anti-Mikrotubulusmittel in ein Reservoir inkorporiert ist, das sich an der künstlichen Herzklappe befindet.

3. Herzklappe nach Anspruch 1 oder Anspruch 2, wobei besagtes Anti-Mikrotubulusmittel in eine Mikrokapsel gekapselt ist, um ein mikroverkapseltes Anti-Mikrotubulusmittel zu bilden.

4. Herzklappe nach Anspruch 1, wobei besagtes Anti-Mikrotubulusmittel in eine Mikrokapsel gekapselt ist, um ein mikroverkapseltes Anti-Mikrotubulusmittel zu bilden und besagtes mikroverkapselte Anti-Mikrotubulusmittel in das Material eingebettet ist, aus dem die künstliche Herzklappe gefertigt ist.

5. Herzklappe nach einem vorhergehenden Anspruch, wobei besagter Nähring ein Polymermaterial umfasst.

6. Herzklappe nach Anspruch 5, wobei besagtes Polymermaterial ein Bauglied umfasst, das aus Kunststoffen, Gummis oder Kombinationen davon ausgewählt ist.

7. Herzklappe nach Anspruch 5, wobei besagtes Polymermaterial ein Gewebe ist.

8. Herzklappe nach Anspruch 7, wobei besagtes Gewebe ein Material umfasst, das ein Bauglied ist, das aus thermoplastischen Polyurethanen (TPU's), Nylons, Polypropylen, Polytetrafluorethylen, Polyestern, Nylonpolymeren, Blockcopolymeren aus einem Polyetherpolymer und einem Polyesterpolymer ausgewählt worden ist und aus Blockcopolymeren aus einem Polyetherpolyol und einem aus der Gruppe bestehend aus Polyamiden, Polyimiden, Polyolefinen, synthetischen Kohlenwasserstoffelastomeren und Naturgummi ausgewählt worden ist.

9. Herzklappe nach Anspruch 8, wobei besagtes Polyester Polyethylenterephthalat (PET) ist.

10. Herzklappe nach Anspruch 8, wobei besagtes Nylon ein Bauglied ist, das aus Nylon-11, Nylon-12 und Kombinationen davon ausgewählt ist.

11. Herzklappe nach Anspruch 8, wobei besagtes Polyolefin ein Bauglied ist, das aus Polyethylenen) (PE) und Polypropylenen (PP) ausgewählt ist.

12. Herzklappe nach einem der Ansprüche 7 bis 11, wobei besagtes Gewebe ein Bauglied ist, das aus einer Kulierware mit Samt, einer Kulierware ohne Samt, einer Kettstuhlware mit Samt, einer Kettstuhlware ohne Samt, einer Webstruktur mit Samt, einer Webstruktur ohne Samt und Kombinationen davon ausgewählt ist.

13. Herzklappe nach einem der Ansprüche 7 bis 12, wobei besagtes Gewebe ein Melangegarn umfasst, das zumindest zwei Polymerkomponenten umfasst.

14. Herzklappe nach Anspruch 13, wobei besagtes Melangegarn Polyester mit Polypropylengarn umwickelt umfasst.

15. Herzklappe nach einem vorhergehenden Anspruch, wobei besagtes Anti-Mikrotubulusmittel ein Bauglied ist, das aus Taxan, Taxanderivaten und Kombinationen davon ausgewählt ist.

16. Herzklappe nach einem vorhergehenden Anspruch, die weiter eine Beschichtungslage umfasst.

17. Herzklappe nach einem der Ansprüche 3 bis 16, wobei besagte Beschichtung über besagte Mikrokapsel geschichtet ist.

18. Herzklappe nach Anspruch 16 oder Anspruch 17, wobei besagte Beschichtung ein Bauglied ist, das aus biologisch erodierbaren Beschichtungen, Hydrogel-Beschichtungen, thermoreversiblen Beschichtungen, biologisch resorbierbaren Beschichtungen und Kombinationen davon ausgewählt ist.

19. Herzklappe nach einem der Ansprüche 3 bis 18, wobei besagte Mikrokapsel aus einem Material gefertigt ist, das im besagten Wirt Erosion unterzogen wird, wodurch kontrollierte Freigabe des besagten eingekapselten Anti-Mikrotubulusmittels aus besagten Mikrokapseln bereitgestellt wird.

20. Herzklappe nach Anspruch 19, wobei besagte Mikrokapsel eine Natriumalginathülle umfasst.

## Revendications

1. Une valvule cardiaque prothétique résistante à une prolifération tissulaire suite à l'implantation de ladite valvule cardiaque prothétique dans un hôte, ladite valvule cardiaque comprenant un anneau de suture et un composant d'hébergement renfermant un composant de valvule, selon laquelle un agent antimicrotubule, en une quantité suffisante pour empêcher une prolifération cellulaire, est incorporé dans l'anneau de suture.

2. Valvule cardiaque selon la Revendication 1, ledit agent antimicrotubule étant incorporé dans un réservoir localisé sur la valvule cardiaque prothétique.

3. Valvule cardiaque selon la Revendication 1 ou la Revendication 2, ledit agent antimicrotubule étant encapsulé dans une microcapsule de manière à former un agent antimicrotubule microencapsulé.

4. Valvule cardiaque selon la Revendication 1, ledit agent antimicrotubule étant encapsulé dans une microcapsule de manière à former un agent antimicrotubule microencapsulé et ledit agent antimicrotubule microencapsulé étant imbriqué dans le matériau à partir duquel la valvule cardiaque prothétique est fabriquée.

5. Valvule cardiaque selon l'une quelconque des revendications précédentes, ledit anneau de suture renfermant un matériau polymérique.

6. Valvule cardiaque selon la Revendication 5, ledit matériau polymérique renfermant un composant sélectionné parmi les plastiques, les caoutchoucs et des combinaisons de ceux-ci.

7. Valvule cardiaque selon la Revendication 5, ledit matériau polymérique étant un tissu.

8. Valvule cardiaque selon la Revendication 7, ledit tissu renfermant un matériau qui est un composant choisi parmi les polyuréthanes thermoplastiques (TPUs), les nylons, le polypropylène, le polytétrafluoroéthylène, les polyesters, les polymères de nylon, les copolymères blocs d'un polymère de polyéther et d'un polymère de polyester, et les copolymères blocs d'un polyol de polyester et un composant sélectionné parmi le groupe constitué de polyamides, polyimides, polyoléfines, élastomères d'hydrocarbone synthétique et caoutchouc naturel.

9. Valvule cardiaque selon la Revendication 8, ledit polyester étant du téréphtalate de polyéthylène (PET).

10. Valvule cardiaque selon la Revendication 8, ledit nylon étant un composant sélectionné parmi le nylon-11, le nylon-12 et une combinaison de ceux-ci.

11. Valvule cardiaque selon la Revendication 8, ladite polyoléfine étant un composant sélectionné parmi les polyéthylènes (PE) et les polypropylènes (PP).

12. Valvule cardiaque selon l'une des Revendications 7 à 11, ledit tissu étant un composant choisi parmi une trame tricotée avec un velours, une trame tricotée sans un velours, une chaîne tricotée avec un velours, une chaîne tricotée sans un velours, une structure de contexture avec un velours, une structure de contexture sans un velours et une combinaison de celles-ci.

13. Valvule cardiaque selon l'une quelconque des Revendications 7 à 12, ledit tissu comprenant un fil de combinaison comprenant au moins deux composants polymériques.

14. Valvule cardiaque selon la Revendication 13, ledit fil de combinaison comprenant du polyester enveloppé d'un fil de polypropylène.

15. Valvule cardiaque selon une quelconque revendication précédente, ledit agent antimicrotubule étant un composant sélectionné parmi la taxane, les dérivés de la taxane et une combinaison de ceux-ci.

16. Valvule cardiaque selon une quelconque revendication précédente, comportant en outre une couche de revêtement.

17. Valvule cardiaque selon l'une quelconque des Revendications 3 à 16, ledit revêtement étant disposé en couches sur ladite microcapsule.

18. Valvule cardiaque selon la Revendication 16 ou la Revendication 17, ledit revêtement étant un composant sélectionné parmi des revêtements bioérodables, des revêtements d'hydrogel, des revêtements thermoréversibles, des revêtements biorésorbables et une combinaison de ceux-ci.

19. Valvule cardiaque selon l'une quelconque des Revendications 3 à 18, ladite microcapsule étant fabriquée à partir d'un matériau subissant une érosion dans ledit hôte, assurant ainsi un relarguage contrôlé dudit agent antimicrotubule encapsulé à partir desdites microcapsules.

20. Valvule cardiaque selon la Revendication 19, ladite microcapsule comportant une enveloppe d'alginate de sodium.
